# EUROPÄISCHE PATENTSCHRIFT

(11) **EP 0 913 394 B1**
(45) Veröffentlichungstag und Bekanntmachung des Hinweises auf die Patenterteilung: **21.12.2005**
(21) Anmeldenummer: 98120019.9
(22) Anmeldetag: 22.10.1998
(51) Int. Cl.: C07D 233/68

(54) **Verfahren zur Herstellung von Formylimidazolen**
Process for the production of formylimidazoles
Procédé pour la préparation de formylimidazoles

(30) Priorität: 29.10.1997 CH 250497
(43) Veröffentlichungstag der Anmeldung: 06.05.1999
(73) Patentinhaber: LONZA A.G., CH-4002 Basel (CH)
(72) Erfinder: Heveling, Josef Dr., 3904 Naters (CH); Wellig, Alain, 3986 Ried-Mörel (CH)
(74) Vertreter: Winter, Brandl, Fürniss, Hübner Röss, Kaiser, Polte Partnerschaft Patent- und Rechtsanwaltskanzlei

(56) Entgegenhaltungen:
- CH-A- 685 496
- S. UESATO ET AL.: "Studies on Monoterpene Glucosides and Related Natural Products. XVVII. Synthesis of 3H- and 2H-labeled Iridodial Derivatives for Studies on the Biosynthesis of Iridoid Glucosides" CHEM. PHARM. BULL., Bd. 30, Nr. 11, 1982, Seiten 3942-3950, XP002092318
- J. D. WHITE ET AL.: "Synthesis of Curacin A: A Powerful Antimitotic from the Cyanobacterium Lyngbya majuscula" J. AM. CHEM. SOC., Bd. 117, Nr. 20, 1995, Seiten 5612-5613, XP002092319
- R. SHIOZAKI ET AL.: "H2O2 Oxidation by Ce(IV) contained weakly-type heteropolyoxometalate for various alcohols" SYNTH. COMM., Bd. 26, Nr. 9, 1996, Seiten 1663-1668, XP002092320
- T. MORIMOTO ET AL.: "Oxidation of Alcohols to Carbonyl Compounds with Peracetic Acid catalysed by Cobalt(III) Acetate" J. CHEM. SOC. PERKIN TRANS. 2,1984, Seiten 1949-1952, XP002092321
- S. P. SRIVASTAVA, V. K. GUPTA: "Kinetics and Mechanism of Ag+ Catalysed Oxidation of Alcohols by Peroxodisulphate Ion in Aqueous Medium" J. INDIAN CHEM. SOC., Bd. 57, Nr. 8, 1980, Seiten 797-799, XP002092322
- H. SUGIMOTO, D. T. SAWYER: "Iron(II)-Induced Activation of Hydroperoxides for the Dehydrogenation and Monooxygenation of Organic Substrates in Acetonitrile" J. AM. CHEM. SOC., Bd. 107, Nr. 20, 1985, Seiten 5712-5716, XP002092323
- P. MÜLLER, H. IDMOUMAZ: "Rhodium-catalyzed oxidation of organic compounds with t-butyl hydroperoxide" J. ORGANOMET. CHEM., Bd. 345, 1988, Seiten 187-199, XP002092324

## Beschreibung

Die vorliegende Erfindung betrifft ein neues Verfahren zur Herstellung von Formylimidazolen der allgemeinen Formel 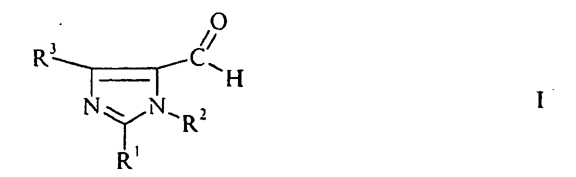 oder 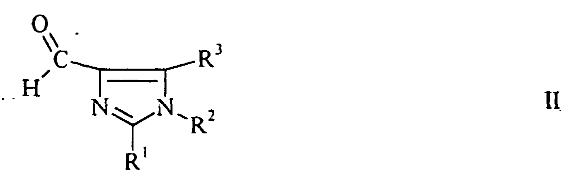 worin R¹ Wasserstoff oder eine gegebenenfalls substituierte Alkylgruppe, R² Wasserstoff, eine gegebenenfalls substituierte Alkyl-, Aryl- oder Arylalkylgruppe und R³ Halogen bedeutet, durch katalytische Oxidation von Hydroxymethylimidazolen der allgemeinen Formel 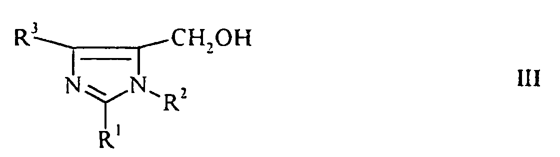 oder 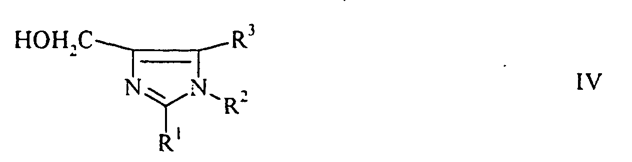 worin R¹, R² und R³ die obengenannte Bedeutung haben.

Formylimidazole sind wichtige Zwischenprodukte z. B. für die Herstellung von pharmazeutischen Wirkstoffen wie Diuretika oder Antihypertonika (WO-A 92/20651). Bisher sind mehrere Verfahren zur Herstellung von Formylimidazolen bekannt.
In der CH-A 685496 wird ein Verfahren beschrieben, bei dem die katalytische Oxidation von Hydroxymethylimidazolen zu Formylimidazolen in Gegenwart von Edelmatallkatalysatoren wie Platinwismuth, Platinschwarz, Platin oder Palladium auf Aktivkohle unter Einblasen von Sauerstoff durchgeführt wird.
Nachteilig bei diesem Verfahren sind die langen Reaktionszeiten von mehreren Stunden und die Bildung von Nebenprodukten.

Die Aufgabe der Erfindung war daher, ein ökonomisches Verfahren zur Herstellung von Formylimidazolen zur Verfügung zu stellen, welches die genannten Nachteile nicht aufweist.

Erfindungsgemäss wird diese Aufgabe durch das Verfahren nach Patentanspruch 1 gelöst. Darin werden Hydroxymethylimidazole der allgemeinen Formel 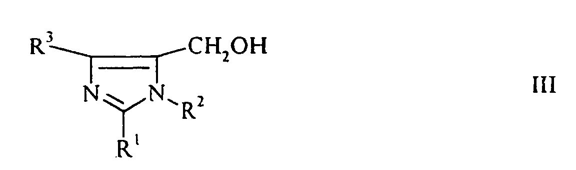 oder 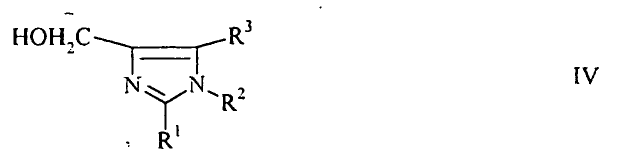 worin R¹, R² und R³ die obengenannte Bedeutung haben, in Gegenwart eines Edelmetallkatalysators und eines Peroxides zu Formylimidazolen der allgemeinen Formel 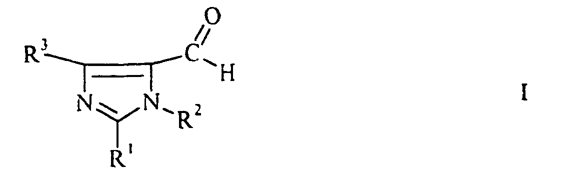 oder 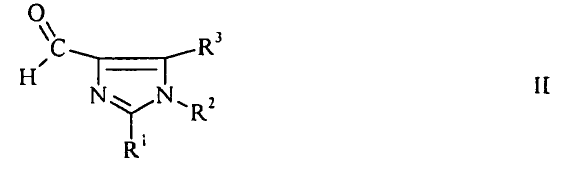 worin R¹, R² und R³ die obengenannte Bedeutung haben, katalytisch oxidiert.

R¹ und R² haben unabhängig voneinander die Bedeutung von Wasserstoff oder einer gegebenenfalls substituierten Alkylgruppe, insbesondere einer geradkettigen oder verzweigten, C₁₋₆-Alkylgruppe Namentlich erwähnt seien Methyl, Ethyl, Propyl, Isopropyl, Butyl, Isobutyl, tert-Butyl, Pentyl und seine Isomeren sowie Hexyl und seine Isomeren. R² kann ausserdem gegebenenfalls substituiertes Aryl oder Arylalkyl, insbesondere Phenyl oder Phenylalkyl, bedeuten, wobei unter Phenylalkyl vorzugsweise Phenyl-C₁₋₆-alkyl, besonders bevorzugt Benzyl verstanden wird. Zweckmässige Substituenten der Alkylgruppen oder der Aromaten der Arylfunktion sind z. B. Halogen, Amino, Alkylamino, Dialkylamino, Alkoxy oder Hydroxy, wobei Alkyl bevorzugt wie oben C₁₋₆-Alkyl und Alkoxy bevorzugt C₁₋₆-Alkoxy, beispielsweise Methoxy oder Ethoxy, bedeutet. Unter Halogen ist hier und im folgenden Fluor, Chlor. Brom oder Jod zu verstehen. Besonders bevorzugt hat R¹ die Bedeutung von Butyl und R² die Bedeutung von Wasserstoff.

R³ hat die Bedeutung von Halogen. Bevorzugte Bedeutung von R³ ist Chlor.

Hydroxymethylimidazole als Ausgangsverbindungen können auf einfache Weise z. B. gemäss der Vorschrift nach der WO-A 92/20651 oder gemäss E. F. Godefroi et al., Trav. Chim. Receuil Pays-Bas, 91, 1383 (1972), hergestellt werden.

Als Edelmetallkatalysator kann Platin, Palladium, Rhodium oder Gold eingesetzt werden. Zweckmässig wird das Edelmetall in Kombination mit Metallen wie beispielsweise Wismuth, Blei, Cerium oder Indium als zweite Komponente eingesetzt. Bevorzugt werden Platin/Wismut- oder Platin/Blei-Katalysatoren eingesetzt.
Der Edelmetallkatalysator wird als solcher oder gebunden an ein Trägermaterial wie z. B. Aktivkohle, Siliciumdioxid, Aluminiumoxid, Silicium-Aluminiumoxid, Zirkonoxid oder Titanoxid eingesetzt. Bevorzugt wird er gebunden an Aktivkohle eingesetzt.
Edelmetallkatalysatoren, welche an Aktivkohle gebunden sind, sind im Handel, z. B. von Degussa, erhältlich.
Der Anteil des an ein Trägermaterial gebundenen Edelmetalles beträgt zweckmässig zwischen 0,1 und 15 Gew.-%, vorzugsweise zwischen 0,5 und 7 Gew.-%, bezogen auf das Trägermaterial.
Der Edelmetallkatalysator wird vorzugsweise in einer Menge von 0,05 bis 1,0 Mol-% auf Edelmetallbasis, bezogen auf das Hydroxymethylimidazol der allgemeinen Formel III oder IV, besonders bevorzugt in einer Menge von 0,1 bis 0,4 Mol-% auf Edelmetallbasis, bezogen auf das Hydroxymethylimidazol der allgemeinen Formel III oder IV, eingesetzt.
Als Peroxide werden organische oder anorganische Peroxide eingesetzt. Gut geeignet sind beispielsweise Wasserstoffperoxid, Perborate, eine Percarbonsäure, tert-Butylhydroperoxid, Cumolhydroperoxid, Perbenzoesäure, m-Chlorperbenzoesäure, Monoperphthalsäure oder Peressigsäure. Besonders gut geeignet ist Wasserstoffperoxid, welches zweckmässig als 10 - 30 %-ige wässerige Lösung eingesetzt wird.

Zweckmässig erfolgt die katalytische Oxidation in Gegenwart von Wasser, einem mit Wasser mischbaren polaren Lösungsmittel, einem mit Wasser nicht mischbaren unpolaren Lösungsmittel oder Mischungen davon, in alkalischem Milieu.
Gut geeignete mit Wasser mischbare polare Lösungsmittel sind beispielsweise Alkohole oder Carbonsäuren mit 1 bis 6 C-Atomen oder Ketone, wie beispielsweise Aceton oder Methylethylketon.
Geeignete mit Wasser nicht mischbare unpolare Lösungsmittel sind z. B. Isobutylmethylketon oder Essigsäureethylester.

Vorteilhaft werden Mischungen von Wasser und einem mit Wasser mischbaren polaren Lösungsmittel, bevorzugt einem Alkohol, besonders bevorzugt Methanol, eingesetzt. Ebenso vorteilhaft werden Mischungen von Wasser und einem mit Wasser nicht mischbaren unpolaren Lösungsmittel, besonders bevorzugt Isobutylmethylketon, eingesetzt. Es hat sich als vorteilhaft erwiesen, wenn das alkalische Milieu durch Zugabe eines Alkalihydroxids, eines Alkalicarbonats oder eines Alkaliacetats zum Reaktionsgemisch erhalten wird. Alkalihydroxid wird vorzugsweise im Verhältnis 1 : 0,05 bis 1,2 vorzugsweise 1 : 0,1 bis 1, bezogen auf die eingesetzte Molmenge des Hydroxymethylimidazols der allgemeinen Formel III oder IV eingesetzt.

Die katalytische Oxidation erfolgt zweckmässig bei einer Temperatur von 20 - 120 °C, vorteilhaft bei 50 - 80°C.

Nach einer üblichen Dosierungszeit des Peroxids von ungefahr 1 Stunde kann, nach genügender Nachreaktionszeit, die Verbindung der allgemeinen Formeln I oder II auf fachmännisch übliche Weise isoliert werden

Die Isolierung des Produktes erfolgt zweckmässig, abhängig vom Lösungsmittelsystem, entweder durch Kristallisation und Filtration oder durch Extraktion mit einem geeigneten Lösungsmittel.

Der verwendete Katalysator kann ohne Aktivitätsverlust mehrmals verwendet werden.

### Beispiele:

### Beispiel 1

### Verfahren zur Herstellung von 2-n-Butyl-4-chlor-5-formylimidazol

4,0 g 2-n-Butyl-4-chlor-5-hydroxymethylimidazol, 21,5 ml 1N NaOH und 13,6 ml Methanol wurden vorgelegt und auf 60 °C aufgeheizt. Es entstand eine Lösung. 0,6g 5 % Pt-5% Bi/C (Degussa, 60 % H₂O enthaltend) wurden zugesetzt. Bei 60 - 62 °C wurden in 60 min 4,2g 20 %-ige wässerige H₂O₂-Lösung zugetropft. Man liess 15 min nachreagieren; dann wurde das Reaktionsgemisch abfiltriert und der Katalysator mit 5 ml Methanol nachgewaschen. Das Filtrat stellte man mit 32 %-iger HCl von pH 12,4 auf pH 7,5. Dieses Gemisch wurde mit 15 ml H₂O versetzt und am Rotavapor teilweise eingedampft (Abziehen von Methanol). Das Gemisch liess man dann unter guter Rührung abkühlen, und die entstandene weisse Suspension wurde bei 20 °C filtriert. Der Filterkuchen wurde mit 5 ml H₂O nachgewaschen und getrocknet. Man erhielt 3,4 g weiss bis leicht-gelbliche Substanz. Diese enthielt nach ¹H-NMR 32,6 mol-% 2-n-Butyl-4-chlor-5-hydroxymethylimidazol und 67,3 mol-% 2-n-Butyl-4-chlor-5-formylimidazol.
¹HNMR-Daten vom 2-n-Butyl-4-chlor-5-formylimidazol:

| | |
|---|---|
| ¹H-NMR (DMSO-_{d16}, 400 MHz) δ : | 13,3 (1 H, bs); |
| | 9,6 (1 H, s); |
| | 2,64 (2 H, t); |
| | 1,63 (2 H, m); |
| | 1,27 (2 H, m); |
| | 0,88 (3 H, t). |

### Beispiel 2

### Verfahren zur Herstellung von 2-n-Butyl-4-chlor-5-formylimidazol

4,0 g 2-n-Butyl-4-chlor-5-hydroxymethylimidazol, 4 ml 1N NaOH, 6 ml H₂O und 12,6 ml Methanol wurden vorgelegt und auf 60 °C aufgeheizt. Es entstand eine Lösung. 0,6g 5 % Pt-5 % Bi/C (Degussa, 60 % H₂O enthaltend) wurden zugesetzt.

Bei 60 - 62 °C wurden in 60 min 4,2 g 20 %-ige wässerige H₂O₂-Lösung zugetropft. Man liess 15 min nachreagieren; dann wurde das Reaktionsgemisch abfiltriert und der Katalysator mit 5 ml Methanol nachgewaschen. Das Filtrat stellte man mit 32 %-iger HCl von pH 8,4 auf pH 7,5. Dieses Gemisch wurde mit 15 ml H₂O versetzt und am Rotavapor teilweise eingedampft (Abziehen von Methanol). Das Gemisch liess man dann unter guter Rührung abkühlen, und die entstandene weisse Suspension wurde bei 2 °C abfiltriert. Der Filterkuchen wurde mit 5 ml H₂O nachgewaschen und getrocknet. Man erhielt 3,7 g weisse Substanz. Diese enthielt nach ¹H-NMR 36,9 mol-% 2-n-Butyl-4-chlor-5-hydroxymethylimidazol und 63,1 mol-% 2-n-Butyl-4-chlor-5-formylimidazol.

### Beispiel 3

### Verfahren zur Herstellung von 2-n-Butyl-4-chlor-5-formylimidazol

3,0 g 2-n-Butyl-4-chlor-5-hydroxymethylimidazol, 1,2 g 5%Pt-5%Bi/C (Degussa, 60% H₂O enthaltend), 44 g Methylisobutylketon, 1,3 ml 1N NaOH-Lösung und 6,8 g Wasser wurden vorgelegt und unter Rühren auf 59 °C aufgeheizt. Bei 60 - 62 °C wurden in 75 min 4,0 g 20%-ige wässrige H₂O₂-Lösung zugetropft. Man liess 15 min nachreagieren; dann wurde das Reaktionsgemisch abfiltriert. Das Filtrat stellte man mit 10%-iger HCl von pH 10,4 auf pH 7,0. Dann gab man das Gemisch in einen Scheidetrichter, die wässerige Phase wurde abgetrennt und mit 10 ml Methylisobutylketon nachextrahiert. Die beiden organischen Phasen wurden vereinigt und am Rotavapor eingeengt. Eindampfrückstand: 3,1 g weisses Kristallisat. Nach ¹H-NMR erhielt man 93,5 mol-% 2-n-Butyl-4-chlor-5-formylimidazol und 6,5 mol-% 2-n-Butyl-4-chlor-5-hydroxymethylimidazol.

## Patentansprüche

1. Verfahren zur Herstellung von Formylimidazolen der allgemeinen Formel 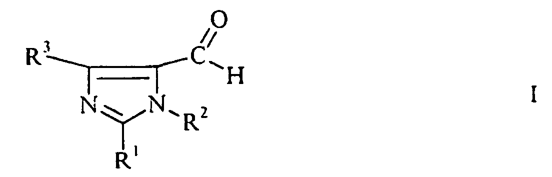 oder 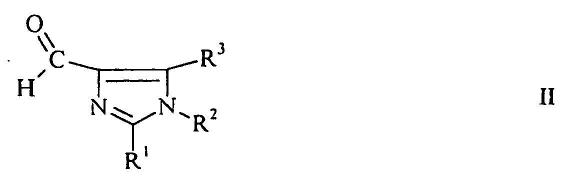 worin R¹ Wasserstoff oder eine gegebenenfalls substituierte Alkylgruppe, R² Wasserstoff, eine gegebenenfalls substituierte Alkyl-, Aryl- oder Arylalkylgruppe und R³ Halogen bedeutet, durch katalytische Oxidation von Hydroxymethylimidazolen der allgemeinen Formel 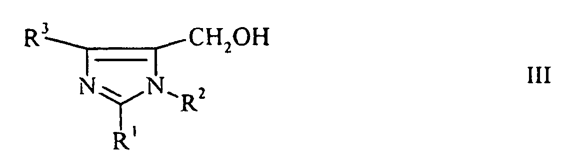 oder 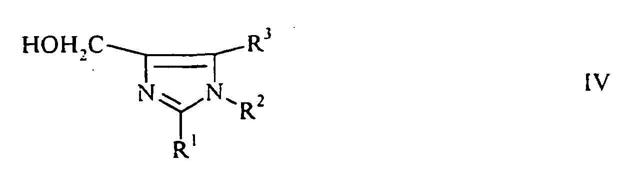 worin R¹, R² und R³ die obengenannte Bedeutung haben, in Gegenwart eines Edelmetallkatalysators, **dadurch gekennzeichnet, dass** die katalytische Oxidation in Gegenwart eines Peroxides erfolgt.

2. Verfahren nach Anspruch 1, **dadurch gekennzeichnet, dass** R¹ eine Butylgruppe ist.

3. Verfahren nach Anspruch 1 oder 2, **dadurch gekennzeichnet, dass** R² Wasserstoff ist.

4. Verfahren nach einem der Ansprüche 1 bis 3, **dadurch gekennzeichnet, dass** der Edelmetallkatalysator ein Platin/Wismut-Katalysator oder ein Platin/Blei-Katalysator ist.

5. Verfahren nach einem der Ansprüche 1 bis 4, **dadurch gekennzeichnet, dass** das Peroxid Wasserstoffperoxid ist.

6. Verfahren nach einem der Ansprüche 1 bis 5, **dadurch gekennzeichnet, dass** die katalytische Oxidation in Gegenwart von Wasser, einem mit Wasser mischbaren polaren Lösungsmittel, einem mit Wasser nicht mischbaren unpolaren Lösungsmittel oder Mischungen davon, in alkalischem Milieu durchgeführt wird.

7. Verfahren nach Patentanspruch 6, **dadurch gekennzeichnet, dass** das alkalische Milieu durch Zugabe eines Alkalihydroxids, Alkalicarbonats oder Alkaliacetats zum Reaktionsgemisch erhalten wird.

8. Verfahren nach einem der Ansprüche 1 bis 7, **dadurch gekennzeichnet, dass** die Umsetzung bei einer Temperatur von 20 - 120 °C durchgeführt wird.

## Claims

1. A process for the preparation of formylimidazoles of the general formula 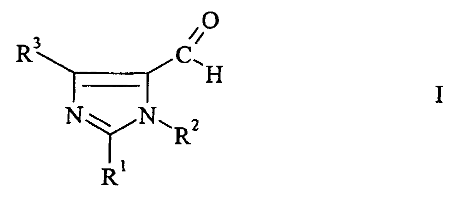 or 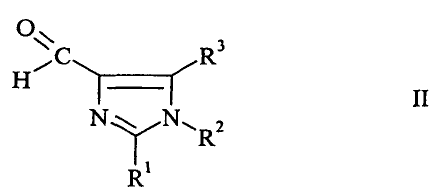 wherein R¹ is hydrogen or an optionally substituted alkyl group, R² is hydrogen, an optionally substituted alkyl, aryl or aralkyl group and R³ is halogen, which comprises catalytically oxidizing of a hydroxymethylimidazole of the general formula 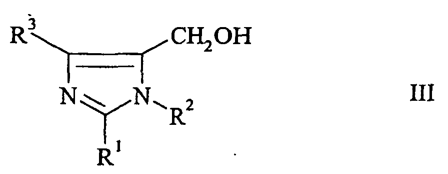 or 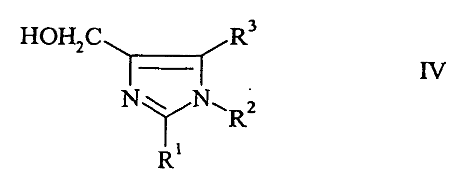 wherein R¹, R² and R³ are as defined above, in the presence of a noble-metal catalyst, **characterized in that** the catalytic oxidation is carried out in the presence of a peroxide.

2. The process of claim 1, **characterized in that** R¹ is a butyl group.

3. The process of claim 1 or 2, **characterized in that** R² is hydrogen.

4. The process of any of claims 1 to 3, **characterized in that** the noble-metal catalyst is a platinum/bismuth catalyst or a platinum/lead catalyst.

5. The process of any of claims 1 to 4, **characterized in that** the peroxide is hydrogen peroxide.

6. The process of any of claims 1 to 5, **characterized in that** the catalytic oxidation is carried out in the presence of water, a water-miscible polar solvent, a water-immiscible nonpolar solvent or a mixture thereof, in alkaline medium.

7. The process of any of claims 1 to 6, **characterized in that** the alkaline medium is obtained by adding an alkali metal hydroxide, alkali metal carbonate or alkali metal acetate to the reaction mixture.

8. The process of any of claims 1 to 7, **characterized in that** the reaction is carried out at a temperature of from 20 to 120 °C.

## Revendications

1. Procédé de préparation de formylimidazoles de formules générales 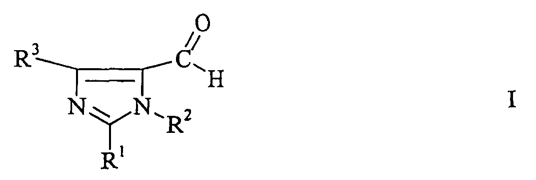 ou 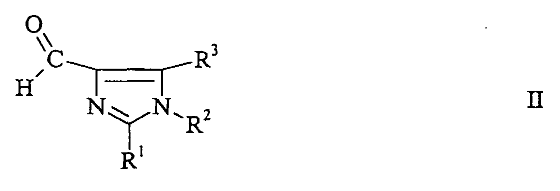 dans lesquelles R¹ représente un hydrogène ou un groupe alkyle éventuellement substitué, R² représente un hydrogène, un groupe alkyle, aryle ou arylalkyle éventuellement substitué et R³ représente un halogène, par oxydation catalytique d'hydroxyméthylimidazoles de formules générales 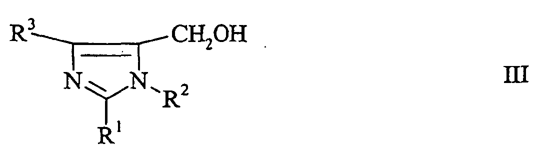 ou 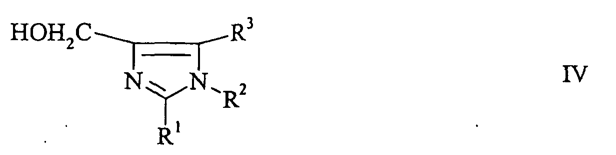 dans lesquelles R¹, R² et R³ présentent la signification susmentionnée, en présence d'un catalyseur à métal noble, **caractérisé en ce que** l'oxydation catalytique est réalisée en présence d'un peroxyde.

2. Procédé selon la revendication 1, **caractérisé en ce que** R¹ est un groupe butyle.

3. Procédé selon la revendication 1 ou 2, **caractérisé en ce que** R² est un hydrogène.

4. Procédé selon l'une quelconque des revendications 1 à 3, **caractérisé en ce que** le catalyseur à métal noble est un catalyseur au platine/bismuth ou un catalyseur au platine/plomb.

5. Procédé selon l'une quelconque des revendications 1 à 4, **caractérisé en ce que** le peroxyde est le peroxyde d'hydrogène.

6. Procédé selon l'une quelconque des revendications 1 à 5, **caractérisé en ce que** l'oxydation catalytique est réalisée en présence d'eau, d'un solvant polaire miscible à l'eau, d'un solvant non polaire non miscible à l'eau ou de leurs mélanges, en milieu alcalin.

7. Procédé selon la revendication 6, **caractérisé en ce que** le milieu alcalin est obtenu par addition d'un hydroxyde de métal alcalin, d'un carbonate de métal alcalin ou d'un acétate de métal alcalin au mélange réactionnel.

8. Procédé selon l'une quelconque des revendications 1 à 7, **caractérisé en ce que** la réaction est réalisée à une température de 20 à 120°C.
